# EUROPEAN PATENT SPECIFICATION

(11) **EP 3 808 260 B1**
(45) Date of publication and mention of the grant of the patent: **14.01.2026**
(21) Application number: 20202214.1
(22) Date of filing: 16.10.2020
(51) Int. Cl.: A61B 5/022, A61B 5/021, A61B 5/00

(54) **SPHYGMOMANOMETER ARM BAND AND CONTROL METHOD THEREOF**
SPHYGMOMANOMETER-ARMBAND UND VERFAHREN ZU DESSEN STEUERUNG
BRASSARD DE SPHYGMOMANOMÈTRE ET SON PROCÉDÉ DE CONTRÔLE

(30) Priority: 18.10.2019 TW 108213812 U
(43) Date of publication of application: 21.04.2021
(73) Proprietor: AViTA Corporation, New Taipei City 24158 (TW)
(72) Inventor: OU, YANG-HSING, 24158 New Taipei City (TW); Lin, LIN, JUI-HSIANG, 24158 New Taipei City (TW)
(74) Representative: Schwerbrock, Florian

(56) References cited:
- EP-A1- 1 609 413
- WO-A1-2013/113333
- CN-U- 201 790 805
- DE-U1- 202011 106 602
- JP-A- H07 124 127

## Description

### BACKGROUND OF THE INVENTION

### 1. Field of the Invention

The present invention relates to a technical field of medical instrument, more particularly to a sphygmomanometer arm band and a control method thereof.

### 2. Description of the Related Art

Blood pressure is an important vital sign for human health and indicates the functional state of the human heart, blood vessels and blood circulation system, and is an important evidence for clinical identification of diseases, examination of medical effects, and prognostic prediction.

With the advancement of science, people's life quality is improving day by day, and the accelerating pace of life makes modern people pay more and more attention to their own health problems. Measuring blood pressure helps people understand their own health and prevent the danger of disease. Therefore, the use of sphygmomanometers has become more and more common.

The arm-type sphygmomanometers, which are convenience in measurement, have become more and more popular. The arm band of the conventional arm-type clamped sphygmomanometer mainly includes a two-piece structure similar to a claw clip, and the two-piece structure includes the hub and spring disposed between two clip parts. When in use, the hub and spring, which are obviously protruded, may cause discomfort on the subject's upper arm in contact. Furthermore, during the process of inflation and deflation of the cuff, the conventional two-piece structure may cause the blood pressure measurement result to be less accurate.

Therefore, the present invention provides a sphygmomanometer arm band and a control method thereof to solve the above-mentioned problems. EP 1 609 413 A1 discloses an electronic sphygmomanometer.

### SUMMARY OF THE INVENTION

An objective of the present invention is to provide a sphygmomanometer arm band for an arm-type sphygmomanometer, and the sphygmomanometer arm band includes a cuff and a clamp. The clamp includes arc-grip part having two opposite ends and configured to elastically clamp on the cuff, and a first arc-shaped force part and a second arc-shaped force part disposed on outer sides of the two opposite ends of the arc-grip part and configured to control a gap between the two opposite ends of the arc-grip part.

The invention is defined by a sphygmomanometer according to claim 1 and a control method according to claim 10. Further embodiments are defined by dependent claims **2-9.**

According to the sphygmomanometer arm band and the control method of the present invention, the configuration of the arc-grip part, the first arc-shaped force part and the second arc-shaped force part can enable the blood pressure measurement operation to be performed more conveniently and make the measurement result more accurate.

### BRIEF DESCRIPTION OF THE DRAWINGS

The structure, operating principle and effects of the present invention will be described in detail by way of various embodiments which are illustrated in the accompanying drawings.
FIG. 1 is a schematic structural view of a first embodiment of a sphygmomanometer arm band, according to the present invention.
FIG. 2 is a sectional view of a sphygmomanometer arm band of FIG. 1 not being applied by an external force, according to the present invention.
FIG. 3 is a schematic structural view of a clamp of a sphygmomanometer arm band of FIG. 1.
FIG. 4 is a side view of the clamp of FIG. 3 not being applied by an external force.
FIG. 5 is a schematic structural view of the clamp of FIG. 3 being applied by an external force.
FIG. 6 is a schematic structural view of a second embodiment of a sphygmomanometer arm band, according to the present invention.
FIGS. 7A and 7B are side view and perspective view of the arc-shaped force part of FIG. 6.

### DETAILED DESCRIPTION OF THE PREFERRED EMBODIMENTS

The following embodiments of the present invention are herein described in detail with reference to the accompanying drawings. These drawings show specific examples of the embodiments of the present invention. These embodiments are provided so that this disclosure will be thorough and complete. It is to be acknowledged that these embodiments are exemplary implementations and are not to be construed as limiting the scope of the present invention in any way, unless required by the claims. These embodiments are provided so that this disclosure is thorough and complete, and fully conveys the concept to those skilled in the art. Regarding the drawings, the relative proportions and ratios of elements in the drawings may be exaggerated or diminished in size for the sake of clarity and convenience. Such arbitrary proportions are only illustrative and not limiting in any way. The same reference numbers are used in the drawings and description to refer to the same or like parts.

Spatially relative terms, such as "central", "lateral", "above", "upper", "beneath", "below", "lower", "left", "right", "vertical", "level", "top", "bottom", "inside", "outside" and the like, may be used herein for ease of description to describe one element or feature's relationship to another element(s) or feature(s) as illustrated in the figures, and may not necessarily mean that an element or a device must be positioned on a specific orientation. It is to be acknowledged that, although the terms 'first', 'second', 'third', and so on, may be used herein to describe various elements, these elements should not be limited by these terms. These terms are used only for the purpose of distinguishing one component from another component. Thus, a first element discussed herein could be termed a second element without altering the description of the present disclosure. As used herein, the term "or" includes any and all combinations of one or more of the associated listed items.

It will be acknowledged that when an element or layer is referred to as being "on," "connected to" or "coupled to" another element or layer, it can be fixedly, detachably or integrally, mechanically, electrically, or directly on, connected or coupled to the other element or layer, or intervening elements or layers may be present. In contrast, when an element is referred to as being "directly on," "directly connected to" or "directly coupled to" another element or layer, there are no intervening elements or layers present.

In addition, unless explicitly described to the contrary, the terms "comprise", "include", "have" and variations such as "comprises", "comprising", "includes", "including", "has", and/or "having" used in the present specification will imply the existence of stated shapes, numbers, steps, operations, elements, and/or groups thereof, but does not the exclusion of any other shapes, numbers, steps, operations, elements, and/or groups thereof.

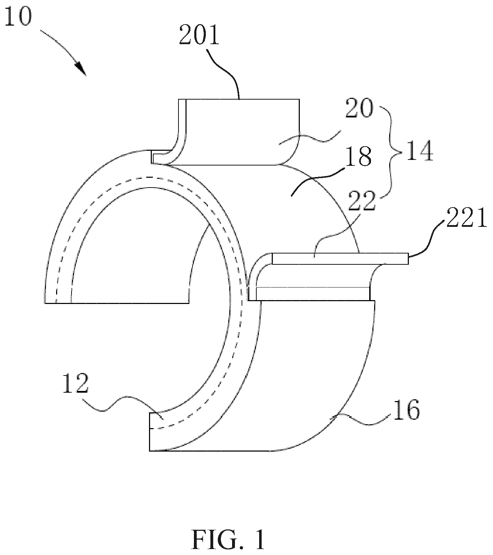

Please refer to FIG. 1, which is a schematic structural view of a sphygmomanometer arm band 10, according to an first embodiment of the present invention. The first embodiment is described for explanation of at least one advantage of the present invention, as shown in FIG. 1, the sphygmomanometer arm band 10 for an arm-type sphygmomanometer includes a cuff 12 and a clamp 14. The cuff 12 includes a cloth cover 16, and the clamp 14 is disposed on the cuff 12 and includes arc-grip part 18, a first arc-shaped force part 20, and a second arc-shaped force part 22. The arc-grip part 18 has two opposite ends 24, and a gap is formed between the two opposite ends 24. Preferably, the gap can have a curvature of 90 degrees, but the present invention is not limited thereto.

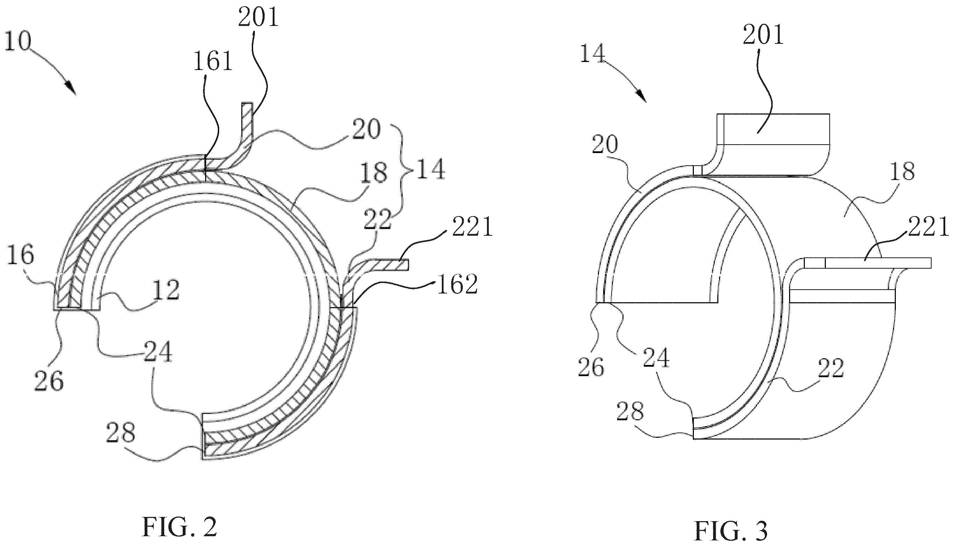

Please refer to FIGS. 1 and 2. FIG. 2 is a sectional view of FIG. 1. As shown in FIG. 2, the clamp 14 includes the arc-grip part 18, the first arc-shaped force part 20 and the second arc-shaped force part 22. In an embodiment of the present invention, the first arc-shaped force part 20 and the second arc-shaped force part 22 are fixedly disposed on outer surfaces of the two opposite ends 24 of the arc-grip part 18, respectively. The arc-grip part 18 is configured to elastically clamp on the cuff 12, to make the cuff 12 tightly attach on a subject's arm. The cloth cover 16 of the cuff 12 is configured to cover the arc-grip part 18, the first arc-shaped force part 20 and the second arc-shaped force part 22, and the cloth cover 16 has a first glove opening 161 and a second glove opening 162 configured to respectively sleeve the two opposite ends 24 of the arc-grip part 18, to make the arc-grip part 18 clamp on the cuff 12. A first handle part 201 of the first arc-shaped force part 20 and a second handle part 221 of the second arc-shaped force part 22 are exposed out of the first glove opening 161 and the second glove opening 162, respectively.

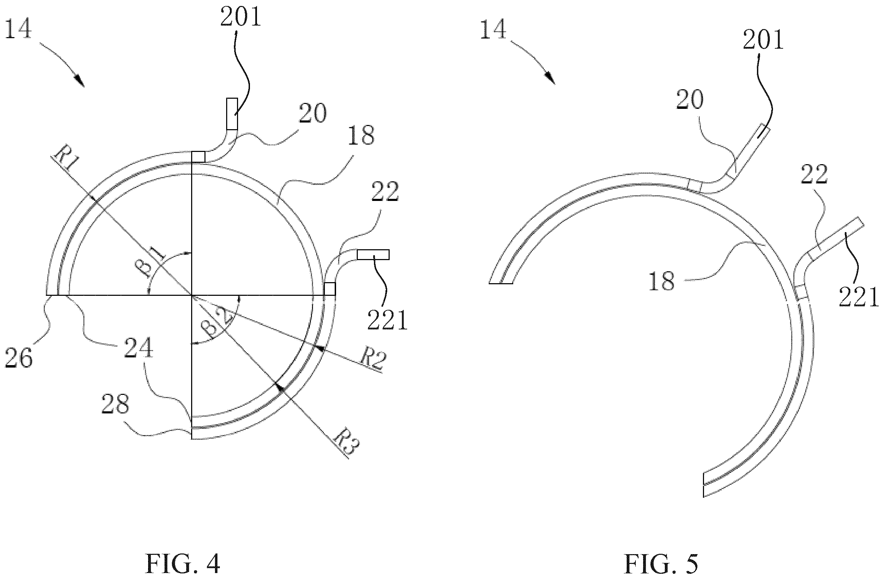

Since the end 26 of the first arc-shaped force part 20 and the end 28 of the second arc-shaped force part 22 are fixed on the two opposite ends 24 of the arc-grip part 18, respectively, the first arc-shaped force part 20 and the second arc-shaped force part 22 can be coupled to the two opposite ends 24 of the arc-grip part 18. The first arc-shaped force part 20 and the second arc-shaped force part 22 have the first handle part 201 and the second handle part 221 extended toward the outer side of the arc-grip part 18, respectively. An interval is formed between the first handle part 201 and the second handle part 221, and distant from the gap between the two opposite ends 24 of the arc-grip part 18. As shown in FIG. 5, a user can apply force on the first handle part 201 of the first arc-shaped force part 20 and the second handle part 221 of the second arc-shaped force part 22, to control the gap between the two opposite ends 24 of the arc-grip part 18, so as to allow the subject's arm to pass through the gap. When the force is removed, as shown in FIG. 4, the arc-grip part 18 can be elastically clamped on the cuff 12, to make the cuff 12 tightly attach on the subject's arm.

However, the manner of connecting the first arc-shaped force part 20 and the second arc-shaped force part 22 and the two opposite ends 24 of the arc-grip part 18 is not limited to the above-mentioned example; for example, the first arc-shaped force part 20 and the second arc-shaped force part 22 can be directly connected to the two opposite ends 24 of the arc-grip part 18 by a locking manner, an adhering manner, or a buckling manner. In an embodiment, the first arc-shaped force part 20, the second arc-shaped force part 22 and the arc-grip part 18 can be formed integrally.

Please refer to FIGS. 1 and 3. FIG. 3 is a schematic structural view of the clamp 14 of the sphygmomanometer arm band 10, according to the present invention. As shown in FIG. 3, the clamp 14 includes the first arc-shaped force part 20, the second arc-shaped force part 22, and the arc-grip part 18. The end 26 of the first arc-shaped force part 20 and the end 28 of the second arc-shaped force part 22 are aligned to the two opposite ends 24 of the arc-grip part 18, respectively, so as to form the gap. The interval is formed between the first handle part 201 of the first arc-shaped force part 20 and the second handle part 221 of the second arc-shaped force part 22 and distant from the gap between the two opposite ends 24. When a force is applied on the first arc-shaped force part 20 and the second arc-shaped force part 22, the first handle part 201 of the first arc-shaped force part 20 and the second handle part 221 of the second arc-shaped force part 22 are moved close to each other, so as to open the gap between the two opposite ends 24 of the arc-grip part 18, to facilitate the subject's arm to put through the gap. After the external force is removed, the curved inner surface of the arc-grip part 18 can make the cuff 12 attach on the subject's arm more closely, so that the measurement result can be more accurate.

Please refer to FIG. 4, which is a side view of the clamp of FIG. 3 not being applied by external force. In this embodiment, a curvature β1 of the first arc-shaped force part 20 is equal to a curvature B1 of the second arc-shaped force part 22, a curvature radius R1 of the first arc-shaped force part 20 is equal to a curvature radius R2 of the second arc-shaped force part 22, and a curvature radius R1 of the first arc-shaped force part 20 is larger than a curvature radius R3 of the arc-grip part 18; however, the present invention is not limited the above-mentioned example, and in other implementations, each of the curvature radius R1 of the first arc-shaped force part 20 and the curvature radius R2 of the second arc-shaped force part 22 can be equal to the curvature radius R3 of the arc-grip part 18; or the curvature radius of one of the first arc-shaped force part 20 and the second arc-shaped force part 22 is larger than the curvature radius R3 of the arc-grip part 18, or the curvature radius of one of the first arc-shaped force part 20 and the second arc-shaped force part 22 is equal to the curvature radius R3 of the arc-grip part 18. In other words, any condition in which the first handle part 201 of the first arc-shaped force part 20 and the second handle part 221 of the second arc-shaped force part 22 can be used to effectively apply force on the two ends 24 of the arc-grip part 18, is adapted to the present invention.

It is to further explain that the arc-grip part 18, the first arc-shaped force part 20 and the second arc-shaped force part 22 can be made by elastic material, to enable the clamp 14 to effectively clamp on the subject's arm, so as to make the cuff 12 attach on the subject's arm. Furthermore, the first arc-shaped force part 20, the second arc-shaped force part 22 and the arc-grip part 18 can be made by the same material.

Please refer to FIGS. 4 and 5. FIG. 5 is a schematic structural view of the clamp 14 of FIG. 3 being applied by external force. As shown in FIG. 5, when an external force is applied on the first handle part 201 of the first arc-shaped force part 20 and the second handle part 221 of the second arc-shaped force part 22, the first arc-shaped force part 20, the second arc-shaped force part 22 and the arc-grip part 18 are deformed. According to the invention, an elastic coefficient of the arc-grip part 18 is less than an elastic coefficient of each of the first arc-shaped force part 20 and the second arc-shaped force part 22, so that the external force applied on the first arc-shaped force part 20 and the second arc-shaped force part 22 can be effectively transferred to the arc-grip part 18, so that the gap between the two ends 24 of the arc-grip part 18 can be opened easier to facilitate a subject to put arm into the clamp 14 when the clamp 14 is in use; however, in examples not covered by the claims, the elastic coefficient of each of the first arc-shaped force part 20 and the second arc-shaped force part 22 can be equal to the elastic coefficient of the arc-grip part 18, or the elastic coefficient of one of the first arc-shaped force part 20 and the second arc-shaped force part 22 can be larger than the elastic coefficient R3 of the arc-grip part 18, or the elastic coefficient of one of the first arc-shaped force part 20 and the second arc-shaped force part 22 can be equal to the elastic coefficient R3 of the arc-grip part 18; in other words, any condition in which the first handle part 201 of the first arc-shaped force part 20 and the second handle part 221 of the second arc-shaped force part 22 can open the gap between the two end 24 of the arc-grip part 18 to facilitate the subject to put arm into the clamp 14, can be adapted to the present disclosure.

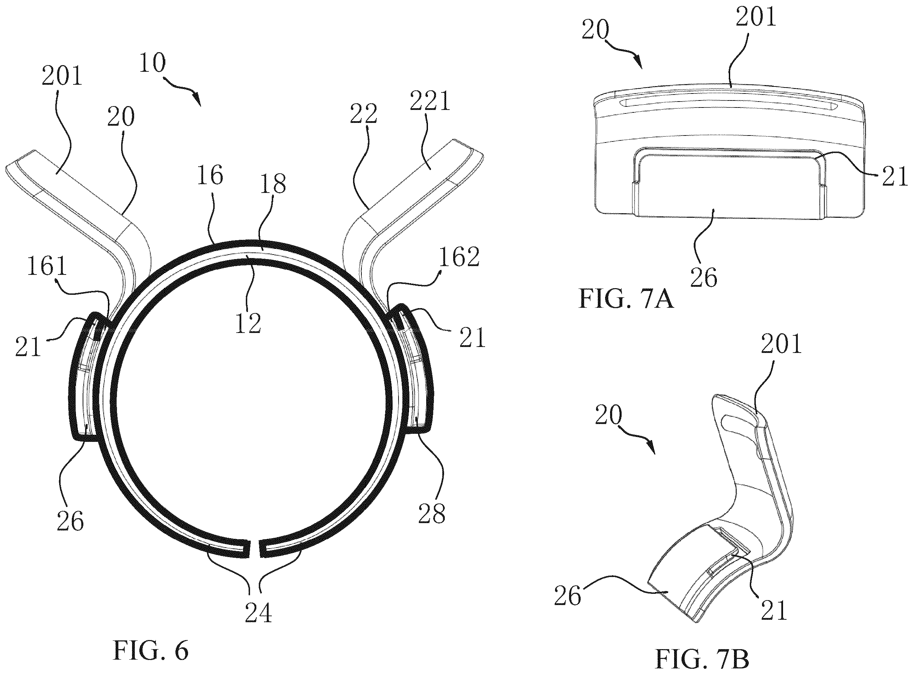

Please refer to FIG. 6, which is a schematic structural view of the sphygmomanometer arm band 10, according to a second embodiment of the present invention. In the second embodiment of the present invention, the sphygmomanometer arm band 10 includes a cuff 12 and a clamp, and the clamp includes arc-grip part 18, a first arc-shaped force part 20 and a second arc-shaped force part 22. The arc-grip part 18 has two opposite ends 24, and a gap is formed between the two opposite ends 24. The cuff 12 includes a cloth cover 16 configured to tightly enclose the arc-grip part 18 of the clamp, so that the cuff 12 can be located on the inner side of the arc-grip part 18. Compared with the first embodiment in which the first arc-shaped force part 20 and the second arc-shaped force part 22 are fixedly disposed on the outer surface of the arc-grip part 18, the first arc-shaped force part 20 and the second arc-shaped force part 22 of the second embodiment of the present invention are detachably mounted on the outer side of the cloth cover 16. The cloth cover 16 enclosing the arc-grip part 18 has a first glove opening 161 and a second glove opening 162 which are located on the outer surface of the arc-grip part 18 and configured to sleeve the end 26 of the first arc-shaped force part 20 and the end 28 of the second arc-shaped force part 22, respectively. With the configuration of tightly enclosing the arc-grip part 18 by the cloth cover 16, an external force can be applied to the first handle part 201 of the first arc-shaped force part 20 and the second handle part 221 of the second arc-shaped force part 22, so as to control the gap between the two opposite ends 24 of the arc-grip part 18 to allow the subject's arm to smoothly pass therethrough. When the external force is removed, the arc-grip part 18 is elastically clamped on the cuff 12, to make the cuff 12 tightly attach with the subject's arm.

In the second embodiment of the present invention, the first arc-shaped force part 20 and the second arc-shaped force part 22, which are detachable, can have the same structures and curve contours. The first arc-shaped force part 20 is taken as example for illustration in following paragraph. FIGS. 7A and 7B show a side view and a perspective view of the first arc-shaped force part 20. The first arc-shaped force part 20 has a first handle part 201 and an end 26 which are formed integrally, and the end 26 of the first arc-shaped force part has the peripheral curvature of the arc-grip part 18, and the first handle part 201 is radially extended from the end 26 of the first arc-shaped force part to the arc-grip part 18. The first arc-shaped force part has a reverse hook structure 21 formed on the end 26, and when the end 26 of the first arc-shaped force part is sleeved into the first glove opening 161 of the cloth cover 16, the reverse hook structure 21 can hook the edge of the first glove opening 161, to maintain the end 26 of the first arc-shaped force part 20 to locate inside the first glove opening 161. Similarly, the second arc-shaped force part 22 having the same structure as the first arc-shaped force part 20 can be sleeved into the second glove opening 162 of the cloth cover 16 by the same way.

In accordance with an embodiment of the present invention, a control method for a sphygmomanometer arm band includes following steps. The cuff 12 is combined with the clamp 14, and the clamp 14 includes the arc-grip part 18, and the first arc-shaped force part 20 and the second arc-shaped force part 22 disposed on outer side of the two opposite ends 24 of the arc-grip part 18, respectively. The arc-grip part 18 is configured to elastically clamp on the cuff 12 and has a gap formed between the two opposite ends 24. The first arc-shaped force part 20 and the second arc-shaped force part 22 can be fixedly disposed on the outer surface of the arc-grip part 18 (such as the first embodiment of the present invention), or the cloth cover 16 can be used to tightly enclose the arc-grip part 18 and the first arc-shaped force part 20 and the second arc-shaped force part 22, which are detachable, are sleeved into the first glove opening 161 and the second glove opening 162 of the cloth cover 16. The first handle part 201 and the second handle part 221 are formed on the first arc-shaped force part 20 and the second arc-shaped force part 22 and extended toward outer side of the arc-grip part 18, respectively. The interval is formed between the first handle part 201 and the second handle part 221 and is distant from the gap of the two opposite ends 24. When the first handle part 201 and the second handle part 221 are controlled by an external force, the first arc-shaped force part 20 and the second arc-shaped force part 22 can control the gap between the two opposite ends 24 of the arc-grip part 18 to facilitate a subject's arm to pass therethrough, so as to make the arc-grip part 18 elastically clamp on the subject's arm and attach the cuff 12 on the arm.

It is to further explain that the sphygmomanometer arm band 10 can include a rubber hose (not shown in figures) and a sphygmomanometer (not shown in figures). The rubber hose is connected to the sphygmomanometer and the cuff 12, and the sphygmomanometer can inflate and deflate the cuff 12 through the rubber hose. The inflation and deflation operations can be performed manually, or the sphygmomanometer can automatically inflate or deflate the cuff 12. In an embodiment, the sphygmomanometer can include a blood pressure calculation component (not shown in figures), and when the inflation and deflation for the cuff 12 is completed, the blood pressure calculation component can calculate the subject's blood pressure value, and show the information of blood pressure value on a display (not shown in figures).

In order to measure the blood pressure, the first arc-shaped force part 20 and the second arc-shaped force part 22 can be forced to open the arc-grip part 18 to directly clamp on the subject's arm, and the sphygmomanometer can be used to inflate and deflate the cuff 12 and calculate the subject's blood pressure value.

According to above-mentioned contents of the sphygmomanometer arm band 10 of the present invention, configuration of the arc-grip part 18, the first arc-shaped force part 20 and the second arc-shaped force part 22 can prevent from compressing the subject's arm to make the subject uncomfortable, and enable the blood pressure measurement operation to be performed more conveniently and make the measurement result more accurate.

The present invention disclosed herein has been described by means of specific embodiments.

## Claims

1. A sphygmomanometer arm band (10) comprising:
a cuff (12); and
a clamp (14) comprising:
arc-grip part (18) having two opposite ends (24) and configured to clamp on the cuff (12);
a first arc-shaped force part (20) and a second arc-shaped force part (22) coupled to the two opposite ends (24) of the arc-grip part (18) and configured to control a gap between the two opposite ends (24) of the arc-grip part (18) such that a subject's arm pass through the gap between the two opposite ends (24), and then inner surface of the arc-grip part (18) elastically clamps on the arm, wherein when an external force is applied on the first arc-shaped force part (20) and the second arc-shaped force part (22), the first arc-shaped force part (20), the second arc-shaped force part (22) and the arc-grip part (18) are deformed;
**characterized in that**
an elastic coefficient of the arc-grip part (18) is less than an elastic coefficient of each of the first arc-shaped force part (20) and the second arc-shaped force part 22, so that the external force applied on the first arc-shaped force part (20) and the second arc-shaped force part (22) can be effectively transferred to the arc-grip part (18).

2. The sphygmomanometer arm band according to claim 1, **characterized in that** the first arc-shaped force part (20) and the second arc-shaped force part (22) have a first handle part (201) and a second handle part (221) extended toward an outer side of the arc-grip part (18), respectively, and an interval is formed between the first handle part (201) and the second handle part (221), and the interval is distant from the gap of the two opposite ends (24).

3. The sphygmomanometer arm band according to claim 2, **characterized in that** when an external force is applied on the first handle part (201) and the second handle part (221), the first arc-shaped force part (20) and the second arc-shaped force part (22) control the gap between the two opposite ends (24) of the arc-grip part (18).

4. The sphygmomanometer arm band according to claim 1, **characterized in that** a curvature radius of the arc-grip part (18) is less than or equal to a curvature radius of one of the first arc-shaped force part (20) and the second arc-shaped force part (22).

5. The sphygmomanometer arm band according to claim 1, **characterized in that** the arc-grip part (18) is made by elastic material.

6. The sphygmomanometer arm band according to claim 5, **characterized in that** an elastic coefficient of the arc-grip part (18) is less than or equal to an elastic coefficient of one of the first arc-shaped force part (20) and the second arc-shaped force part (22).

7. The sphygmomanometer arm band according to claim 1, **characterized in that** an elastic coefficient of the arc-grip part (18) is less than or equal to an elastic coefficient of one of the first arc-shaped force part (20) and the second arc-shaped force part (22).

8. The sphygmomanometer arm band according to claim 1, **characterized in that** the cuff (12) comprises a cloth cover (16) having a first glove opening (161) and a second glove opening (162), the first glove opening (161) and the second glove opening (162) of the cloth cover (16) are configured to sleeve the two ends (26, 28) of the first arc-shaped force part (20) and the second arc-shaped force part (22), respectively, and make the arc-grip part (18) clamp on the cuff (12), and the first handle part (201) of the first arc-shaped force part (20) and the second handle part (221) of the second arc-shaped force part (22) are exposed out of the first glove opening (161) and the second glove opening (162), respectively.

9. The sphygmomanometer arm band according to claim 1, **characterized in that** the gap between the two opposite ends (24) of the arc-grip part (18) has a curvature of 90 degrees.

10. A control method applied to the sphygmomanometer arm band of claim 1, and the control method comprising:
providing a first handle part (201) and a second handle part (221) formed on the first arc-shaped force part (20) and the second arc-shaped force part (22) and extended toward an outer side of the arc-grip part (18), respectively, wherein the first arc-shaped force part (20) and the second arc-shaped force part (22) are coupled to two opposite ends (24) of the arc-grip part (18), respectively, an interval is formed between the first handle part (201) and the second handle part (221), and the interval is distant from the gap between the two opposite ends (24); and
when an external force is applied on the first handle part (201) and the second handle part (221), using the first arc-shaped force part (20) and the second arc-shaped force part (22) to make the gap between the two opposite ends (24) of the arc-grip part (18) pass a subject's arm therethrough, and then inner surface of the arc-grip part (18) elastically clamps on the arm via the elastic deformation of the elastic part (18), so as to attach the cuff (12) on the subject's arm, wherein when the external force is applied on the first handle part (201) of the first arc-shaped force part (20) and the second handle part (221) of the second arc-shaped force part (22), the first arc-shaped force part (20), the second arc-shaped force part (22) and the arc-grip part (18) are deformed, **characterized in that**
an elastic coefficient of the arc-grip part (18) is less than an elastic coefficient of each of the first arc-shaped force part (20) and the second arc-shaped force part 22, so that the external force applied on the first arc-shaped force part (20) and the second arc-shaped force part (22) can be effectively transferred to the arc-grip part (18).

## Patentansprüche

1. Blutdruckmessgerät-Armband (10), umfassend:
- eine Manschette (12); und
- eine Klemme (14), umfassend:
einen bogenförmigen Klemmteil (18), die zwei gegenüberliegende Enden (24) aufweist und so gestaltet ist, dass er die Manschette (12) elastisch klemmt; und
einen ersten bogenförmigen Kraftteil (20) und einen zweiten bogenförmigen Kraftteil (22), die mit den beiden gegenüberliegenden Enden (24) des bogenförmigen Klemmteils (18) gekoppelt und dafür gestaltet sind, einen Spalt zwischen den beiden gegenüberliegenden Enden (24) des bogenförmigen Klemmteils (18) so einzustellen, dass ein Arm der Testperson durch den Spalt zwischen den beiden Enden (24) hindurch gesteckt werden kann und die innere Oberfläche des bogenförmigen Klemmteils (18) sodann den Arm elastisch klemmt, wobei sich der erste und der zweite bogenförmige Kraftteil (20, 22) dann verformen, wenn eine externe Kraft auf den ersten und den zweiten bogenförmigen Kraftteil (20, 22) ausgeübt wird,
**dadurch gekennzeichnet,**
**dass** der Elastizitätskoeffizient des bogenförmigen Klemmteils (18) kleiner als der Elastizitätskoeffizient des ersten bogenförmigen Kraftteils (20) und des zweiten bogenförmigen Kraftteils (22) ist, sodass die auf den ersten und den zweiten bogenförmigen Kraftteil (20, 22) ausgeübte externe Kraft effizient auf den bogenförmigen Klemmteil (18) übertragen werden kann.

2. Blutdruckmessgerät-Armband (10) nach Anspruch 1,
**dadurch gekennzeichnet,**
**dass** der erste und der zweite bogenförmige Kraftteil (20, 22) jeweils einen ersten Griffteil (201) und einen zweiten Griffteil (221) aufweisen, die sich zu einer Außenseite des bogenförmigen Klemmteils (18) erstrecken, wobei eine Lücke zwischen dem ersten Griffteil (201) und dem zweiten Griffteil (221) derart ausgebildet ist, dass sie von dem Spalt zwischen den beiden gegenüberliegenden Enden (24) des bogenförmigen Klemmteils (18) entfenrt ist.

3. Blutdruckmessgerät-Armband (10) nach Anspruch 2,
**dadurch gekennzeichnet,**
**dass** der Spalt zwischen den beiden gegenüberliegenden Enden (24) des bogenförmigen Klemmteils (18) dann mittels des ersten und des zweiten bogenförmigen Kraftteils (20, 22) eingestellt wird, wenn eine externe Kraft auf den ersten und den zweiten Griffteil (201, 221) ausgeübt wird.

4. Blutdruckmessgerät-Armband (10) nach Anspruch 1,
**dadurch gekennzeichnet,**
**dass** der Krümmungskoeffizient des bogenförmigen Klemmteils (18) kleiner als oder gleich dem Krümmungskoeffizient eines der beiden bogenförmigen Kraftteile (20, 20) ist.

5. Blutdruckmessgerät-Armband (10) nach Anspruch 1,
**dadurch gekennzeichnet,**
**dass** der bogenförmige Klemmteil (18) aus elastischem Material hergestellt ist.

6. Blutdruckmessgerät-Armband (10) nach Anspruch 5,
**dadurch gekennzeichnet,**
**dass** der Elastizitätskoeffizient des bogenförmigen Klemmteils (18) kleiner als oder gleich dem Elastizitätskoeffizient eines der beiden bogenförmigen Kraftteile (20, 20) ist.

7. Blutdruckmessgerät-Armband (10) nach Anspruch 1,
**dadurch gekennzeichnet,**
**dass** der Elastizitätskoeffizient des bogenförmigen Klemmteils (18) kleiner als oder gleich dem Elastizitätskoeffizient eines der beiden bogenförmigen Kraftteile (20, 20) ist.

8. Blutdruckmessgerät-Armband (10) nach Anspruch 1,
**dadurch gekennzeichnet,**
**dass** die Manschette (12) einen Stoffüberzug (16) umfasst, der eine erste Überzugsöffnung (161) und eine zweite Überzugsöffnung (162) aufweist, die so gestaltet sind, dass sie jeweils die beiden gegenüberliegenden Enden (24) des bogenförmigen Klemmteils 18 umschließen, sodass der bogenförmige Klemmteil 18 die Manschette 12 klemmt; und
**dass** der erste Griffteil (201) des ersten bogenförmigen Kraftteils (20) und der zweite Griffteil (221) des zweiten bogenförmigen Krafteils (22) jeweils aus der ersten Überzugsöffnung (161) bzw. der zweiten Überzugsöffnung (162) herausragen.

9. Blutdruckmessgerät-Armband (10) nach Anspruch 1,
**dadurch gekennzeichnet,**
**dass** der Spalt zwischen den beiden gegenüberliegenden Enden (24) des bogenförmigen Klemmteils (18) eine Krümmung von 90 Grad aufweist.

10. Einstellverfahren, das auf das Blutdruckmessgerät-Armband (10) nach Anspruch 1 angewendet wird, wobei das Einstellverfahren folgende Schritte umfasst:
- Bereitstellen eines ersten Griffteils (201) und einen zweiten Griffteils (221), die jeweils an dem ersten bogenförmigen Kraftteil (20) und dem zweiten bogenförmigen Kraftteil (22) ausgebildet sind und sich zu einer Außenseite des bogenförmigen Klemmteils (18) erstrecken, wobei der erste und der zweite bogenförmigenKraftteil (20, 20) jeweils mit beiden gegenüberliegenden Enden (24) des bogenförmigen Klemmteils (18) gekoppelt sind, wobei eine Lücke zwischen dem ersten Griffteil (201) und dem zweiten Griffteil (221) so ausgebildet ist, dass sie von dem Abstand zwischen den beiden gegenüberliegenden Enden (24) entfernt ist; und
- Ausüben einer externen Kraft auf den ersten Griffteil (201) und den zweiten Griffteil (221) und Formen des Spaltes zwischen den beiden gegenüberliegenden Enden (24) des bogenförmigen Klemmteils (18) mithilfe des ersten bogenförmigen Kraftteils (20) und des zweiten bogenförmigen Kraftteils (22) so, dass der Arm der Testperson durch den Spalt hindurchpasst. Anschließend klemmt die Innenfläche des bogenförmigen Klemmteils (18) den Arm durch die elastische Verformung des bogenförmigen Klemmteils (18) elastisch ein und fixiert so die Manschette (12) am Arm der Testperson. Wird eine äußere Kraft auf den ersten Griffteil (201) des ersten bogenförmigen Kraftteils (20) und den zweiten Griffteil (221) des zweiten bogenförmigen Kraftteils (22) ausgeübt, so verformen sich der erste bogenförmige Kraftteil (20), der zweite bogenförmige Kraftteil (22) und der bogenförmige Klemmteil (18);
**dadurch gekennzeichnet,**
**dass** der Elastizitätskoeffizient des bogenförmigen Klemmteils (18) kleiner als die Elastizitätskoeffizienten des ersten und des zweiten bogenförmigen Kraftteils (20, 22) ist, sodass die auf den ersten und den zweiten bogenförmigen Kraftteil (20, 22) ausgeübte äußere Kraft effektiv auf den bogenförmigen Klemmteil (18) übertragen werden kann.

## Revendications

1. Un brassard pour sphygmomanomètre (10) comprenant :
une manchette (12) et
un élément de serrage (14) comprenant :
une partie adhérence en arc (18) comportant deux extrémités opposées (24) et configurée pour être serrée sur la manchette (12) ;
une première partie d'application de force en forme d'arc (20) et une deuxième partie d'application de force en forme d'arc (22) couplées aux deux extrémités opposées (24) de la partie adhérence en arc (18) et configurées pour contrôler un écartement entre les deux extrémités opposées (24) de la partie adhérence en arc (18), de sorte que le bras d'un sujet passe par l'écartement entre les deux extrémités opposées (24) et que la surface intérieure de la partie adhérence en arc (18) se serre alors élastiquement sur le bras, où, lorsqu'une force extérieure est appliquée sur la première partie d'application de force en forme d'arc (20) et la deuxième partie d'application de force en forme d'arc (22), la première partie d'application de force en forme d'arc (20), la deuxième partie d'application de force en forme d'arc (22) et la partie adhérence en arc (18) sont déformées, **caractérisée en ce qu'**un coefficient d'élasticité de la partie adhérence en arc (18) est inférieur à un coefficient d'élasticité de la première partie d'application de force en forme d'arc (20) et de la deuxième partie d'application de force en forme d'arc (22), de sorte que la force extérieure appliquée sur la première partie d'application de force en forme d'arc (20) et la deuxième partie d'application de force en forme d'arc (22) puisse être efficacement transférée à la partie adhérence en arc (18).

2. Le brassard pour sphygmomanomètre selon la revendication 1, **caractérisé en ce que** la première partie d'application de force en forme d'arc (20) et la deuxième partie d'application de force en forme d'arc (22) comportent une première partie de poignée (201) et une deuxième partie de poignée (221) s'étendant vers un côté extérieur de la partie adhérence en arc (18), respectivement, qu'un intervalle se forme entre la première partie de poignée (201) et la deuxième partie de poignée (221) et que l'intervalle est distant de l'écartement des deux extrémités opposées (24).

3. Le brassard pour sphygmomanomètre selon la revendication 2, **caractérisé en ce que**, lorsqu'une force extérieure est appliquée sur la première partie de poignée (201) et la deuxième partie de poignée (221), la première partie d'application de force en forme d'arc (20) et la deuxième partie d'application de force en forme d'arc (22) contrôlent l'écartement entre les deux extrémités opposées (24) de la partie adhérence en arc (18).

4. Le brassard pour sphygmomanomètre selon la revendication 1, **caractérisé en ce qu'**un rayon de courbure de la partie adhérence en arc (18) est inférieur ou égal à un rayon de courbure de la première partie d'application de force en forme d'arc (20) ou de la deuxième partie d'application de force en forme d'arc (22).

5. Le brassard pour sphygmomanomètre selon la revendication 1, **caractérisé en ce que** la partie adhérence en arc (18) est réalisée dans un matériau élastique.

6. Le brassard pour sphygmomanomètre selon la revendication 5, **caractérisé en ce qu'**un coefficient d'élasticité de la partie adhérence en arc (18) est inférieur ou égal à un coefficient d'élasticité de la première partie d'application de force en forme d'arc (20) ou de la deuxième partie d'application de force en forme d'arc (22).

7. Le brassard pour sphygmomanomètre selon la revendication 1, **caractérisé en ce qu'**un coefficient d'élasticité de la partie adhérence en arc (18) est inférieur ou égal à un coefficient d'élasticité de la première partie d'application de force en forme d'arc (20) ou de la deuxième partie d'application de force en forme d'arc (22).

8. Le brassard pour sphygmomanomètre selon la revendication 1, **caractérisé en ce que** la manchette (12) comporte un revêtement en tissu (16) ayant une première ouverture de gant (161) et une deuxième ouverture de gant (162), la première ouverture de gant (161) et la deuxième ouverture de gant (162) du revêtement en tissu (16) sont configurées pour enserrer les deux extrémités (26, 28) de la première partie d'application de force en forme d'arc (20) et de la deuxième partie d'application de force en forme d'arc (22), respectivement, et pour permettre à la partie adhérence en arc (18) de se serrer sur la manchette (12), et que la première partie de poignée (201) de la première partie d'application de force en forme d'arc (20) et la deuxième partie de poignée (221) de la deuxième partie d'application de force en forme d'arc (22) sont exposées hors de la première ouverture de gant (161) et de la deuxième ouverture de gant (162), respectivement.

9. Le brassard pour sphygmomanomètre selon la revendication 1, **caractérisé en ce que** l'écartement entre les deux extrémités opposées (24) de la partie adhérence en arc (18) présente une courbure de 90 degrés.

10. Une méthode de contrôle appliquée au brassard pour sphygmomanomètre de la revendication 1, la méthode de contrôle consistant à :
fournir une première partie de poignée (201) et une deuxième partie de poignée (221) formées sur la première partie d'application de force en forme d'arc (20) et la deuxième partie d'application de force en forme d'arc (22), et s'étendant vers un côté extérieur de la partie adhérence en arc (18), respectivement, où la première partie d'application de force en forme d'arc (20) et la deuxième partie d'application de force en forme d'arc (22) sont couplées aux deux extrémités opposées (24) de la partie adhérence en arc (18), respectivement, où un intervalle est formé entre la première partie de poignée (201) et la deuxième partie de poignée (221), et où l'intervalle est distant de l'écartement entre les deux extrémités opposées (24), et
lorsqu'une force extérieure est appliquée sur la première partie de poignée (201) et la deuxième partie de poignée (221), la première partie d'application de force en forme d'arc (20) et la deuxième partie d'application de force en forme d'arc (22) sont utilisées pour permettre à l'écartement entre les deux extrémités opposées (24) de la partie adhérence en arc (18) de faire passer le bras d'un sujet, et la surface intérieure de la partie adhérence en arc (18) se serre alors élastiquement sur le bras via la déformation élastique de la partie élastique (18), de façon à fixer la manchette (12) sur le bras du sujet, où, lorsque la force extérieure est appliquée sur la première partie de poignée (201) de la première partie d'application de force en forme d'arc (20) et la deuxième partie de poignée (221) de la deuxième partie d'application de force en forme d'arc (22), la première partie d'application de force en forme d'arc (20), la deuxième partie d'application de force en forme d'arc (22) et la partie adhérence en arc (18) sont déformées, **caractérisé en ce qu'**un coefficient d'élasticité de la partie adhérence en arc (18) est inférieur à un coefficient d'élasticité de la première partie d'application de force en forme d'arc (20) et de la deuxième partie d'application de force en forme d'arc (22), de sorte que la force extérieure appliquée sur la première partie d'application de force en forme d'arc (20) et la deuxième partie d'application de force en forme d'arc (22) puisse être efficacement transférée à la partie adhérence en arc (18).
